# EUROPÄISCHE PATENTANMELDUNG

(11) **EP 1 411 358 A1**
(43) Veröffentlichungstag der Anmeldung: **21.04.2004**
(21) Anmeldenummer: 03021513.1
(22) Anmeldetag: 24.09.2003
(51) Int. Cl.: G01N 33/86, G01N 27/447

(54) **Verfahren zur Bestimmung von Multimeren von Plasmaproteinen**

(30) Priorität: 14.10.2002 DE 10247876
(71) Anmelder: Aventis Behring GmbH, 35002 Marburg (DE)
(72) Erfinder: Hermentin, Peter, 35041 Marburg (DE); Cuesta-Linker, Thomas, 35083 Wetter (DE); Schmidt, Karl-Heinz, 35096 Weimar (Lahn) (DE)

(57) **Zusammenfassung**

Es wird ein Verfahren zur qualitativen und quantitativen Bestimmung von Multimeren von Fibrinogen und des von Willebrand-Faktors durch Gelelektrophorese beschrieben, bei dem man eine den von Willebrand-Faktor (vWF) oder Fibrinogen enthaltende Probe mittels eines kontinuierlichen, homogenen und von Verklumpungen freien Agarose-Gels submarin elektrophoretisch auftrennt und die Multimerenbanden nach einer Western Blot-Analyse durch ein spezifisches Antikörper-Enzym-Konjugat auf der Blotting-Membran immunchemisch oder durch einen geeigneten Farbstoff, vorzugsweise unter Blaufärbung, im Gel sichtbar macht.

## Beschreibung

Gegenstand der Erfindung ist ein Verfahren zur qualitativen und quantitativen Bestimmung der Multimeren von Plasmaproteinen durch Gelelektrophorese.

Es ist bekannt, dass für die Blutgerinnung wichtige Bestandteile des Blutplasma, nicht nur in monomerer Form, sondern auch als multimere Moleküle im Blutplasma vorkommen. Den einzelnen Monomeren kann zum Teil unterschiedliche therapeutische Wertigkeit zugeordnet werden. Beispiele für solche multimeren bildenden therapeutischen Proteine sind Fibrinogen und der von Willebrand-Faktor. Die quantitative und qualitative Bestimmung der Multimeren ist für eine Diagnose der Ursache der Blutgerinnungsstörung einerseits, aber auch zum Erkennen der Qualität eines Blutgerinnungspräparats andererseits von erheblicher Bedeutung. Die Multimeren-Bestimmung des Fibrinogens und des von Willebrand-Faktors verdient deshalb besondere Beachtung.

Fibrinogen hat bei der Blutstillung eine ganz wesentliche Funktion. Unter der Wirkung von Thrombin wird es in Fibrin umgewandelt, das sich zu feinen Fibrinfasern vernetzt, die durch den Faktor XIII stabilisiert werden. Hieraus bildet sich ein Blutpfropf, der für die Blutstillung sorgt. Die Integrität und die Struktur des Fibrinogens ist für seine blutstillende Wirkung von großer Bedeutung.

Obwohl Fibrinogen selbst nicht zur Polymersiation neigt, bilden sich unter geeigneten Bedingungen wie hoher Konzentration, geringer Ionenstärke und langer Inkubation bei 4°C auch Fibrinogen-Multimere ähnlich dem Fibrin. Derartige Fibrinogene lassen sich über SDS-Agarose-Gel-Elektrophorese mit Western Blot und immunchemischer Färbung analysieren und sichtbar machen, wie es in der internationalen Patentanmeldung WO 01/12244 beschrieben ist.

Die Verfahren zur Trennung von Fibrinogen-Multimeren durch SDS-Agarose-Gel-Elektrophorese wurden durch Connaghan et al. (1 ) und Proietti et al. (2) eingeführt. Diese Verfahren wurden dann durch das in der internationalen Patentanmeldung WO 01/12244 beschriebene Verfahren verbessert, in dem die von Raines et al. (3) für die Trennung der von Willebrand-Multimeren beschriebene Methode modifiziert und zur Fibrinogenelektrophorese genutzt wurde. Dabei wurde die horizontale Elektrophorese im wesentlichen nach (3) und die Detektion der Fibrinogen-Multimeren im wesentlichen nach (2) durchgeführt.

Das Elektrophoreseverfahren der WO 01/12244 ist jedoch recht kompliziert sowie zeitaufwendig und erfordert sehr spezielle Erfahrungen. Dabei werden drei verschiedene Gele benötigt, nämlich ein Sammelgel (1% Agarose gelöst in 70 mM Tris, 4 mMol EDTA, 4% SDS), ein Kathodengel (1,6% Agarose gelöst in 100 mM Tris, 150 mM Glycin, 0,1% SDS) und ein Trenngel (2% Agarose gelöst in 200 mM Tris, 100 mM Glycin, 0,4% SDS), die bei einer Temperatur über 50°C gegossen und mindestens 2 Stunden zur Polymerisation stehen gelassen werden müssen. Der Probenauftrag erfolgt mit 0,6 bis 0,9 µl pro Spur. Die Elektrophorese wird bei 600 bis 650 V und bei einer Temperatur von 16°C in einer Flachbettapparatur durchgeführt. Der Laufpuffer wird mit dem Gel über eine Länge von 4 bis 8 Filterpapieren verbunden, die in den Elektrodenpuffer eintauchen.

Die Herstellung dreier verschiedener Agarose-Gele erfordert eine erhebliche Erfahrung, weil der Quellvorgang der Agarose nahe der Siedehitze des Wassers durchgeführt werden muss. Dabei kommt es darauf an, den optimalen Zeitpunkt zu finden, zu dem einerseits die Agarose so weit gelöst, dass keine Klumpen (Feststoffreste) mehr vorhanden sind und andererseits noch keine Agarose am oberen, kälteren Glasrand als Präzipitat erneut niedergeschlagen ist. Auch das Gießen des Gels erfordert eine spezielle Geschicklichkeit, insbesondere das Einbringen der Auftragstaschen. Das Entfernen des zur Bildung der Auftragstaschen erforderlichen Kammes aus dem abgekühlten, gehärteten Gel kann leicht zu einer Verletzung des Agarose-Gels und damit zur Unbrauchbarkeit des gegossenen Gels führen.

Ähnliche Schwierigkeiten treten auch bei den bisher üblichen Verfahren zur Auftrennung der Multimeren des von Willebrand-Faktors (vWF) auf.

Der funktionelle von Willebrand-Faktor (vWF), ein Glykoprotein, zirkuliert im Blutkreislauf mit unterschiedlicher Molekulargewichtsverteilung, den sog. Multimeren, wobei die Multimeren eine Molekulargewichtsverteilung von 500 kD bis hin zu 20.000 kD aufweisen können. Die kleinste Einheit ist hierbei das Dimer mit einem Molekulargewicht von ca. 550 kD; es besteht aus zwei Monomeren, die durch Disulfidbrücken miteinander verbunden sind. Aus diesen Dimeren entstehen durch weitere Disulfidverknüpfungen Polymere, sog. Multimere, mit einem Molekulargewicht bis zu 20.000 kD.

Das klinische Erscheinungsbild der von Willebrand-Erkrankung ist sehr heterogen und reicht von leichten Verläufen wie dem Typ 1 bis hin zu schwersten Mangelzuständen mit spontan auftretenden Blutungen beim Typ 3. Ursächlich für die Blutungsneigung sind quantitative und/oder qualitative Beeinträchtigungen der beiden Hauptfunktionen des vWF, der Vermittlung der Adhäsion von Thrombozyten untereinander und an die verletzte Gefäßwand sowie die Stabilisierung des Gerinnungsfaktors Faktor VIII. Somit können sowohl die frühe primäre als auch die später einsetzende sekundäre Hämostase gestört sein.

Bei einer sekundären Blutgerinnungsstörung, bedingt durch einen Faktor VIII-Mangel, ist der Faktor VIII jedoch nicht zwangsläufig vermindert, sondern die Blutgerinnungsstörung kann auch Ausdruck eines quantitativen und/oder qualitativen Defektes des von Willebrand-Faktors sein.

Bedingt durch die Komplexität der Gerinnungsstörung ist die Diagnose und Einteilung der von Willebrand-Erkrankung in seine verschiedenen Schweregrade eine Herausforderung an jedes Gerinnungslabor. Für den Patienten selbst hat die exakte Diagnosestellung, d.h. die Bestimmung des Untertyps, wichtige Folgen, denn es kann nur dann eine optimale Behandlung ausgewählt werden und für den Fall eines genetischen Defekts eine genauere Beratung erfolgen. So kann bei schweren von Willebrand-Erkrankungen vom Typ 3 und vielen der Typ 2-Formen eine Behandlung mit Desmopressin, das bei Typ 1-Erkrankungen hervorragend wirksam ist, nicht ausreichen oder sogar kontraindiziert sein, so dass hier ein vWF-haltiges Faktor VIII-Konzentrat gegeben werden muss. Dabei zeigen insbesondere Faktor VIII-Konzentrate mit großen vWF-Multimeren eine gute therapeutische Wirksamkeit.

Der vWF ist kein Eiweiß der plasmatischen Gerinnung (wie Faktor VIII oder IX), sondern wirkt über eine Brückenbildung zwischen verletzter Gefäßwand und den Blutplättchen als ein wichtiger Bestandteil der primären Blutstillung und als sog. Klebstoff für die Blutplättchen. Weiterhin stabilisiert er den Faktor VIII, der ohne Bindung an den vWF im Blut nur sehr kurze Zeit zirkulieren kann, da er sonst rasch abgebaut wird.

Erfindungsgemäß erhält man Informationen über Strukturveränderungen des vWF-Moleküls durch Darstellung der Multimeren mittels einer SDS-Agarose-Gel-Elektrophorese mit Western Blot-Immunostain Analyse (SAGE-WISA) oder durch Coomassie Blue-Färbung des Gels (SAGE-CB). Die elektrophoretische Darstellung der Multimeren erlaubt dann Aussagen über strukturelle Besonderheiten. Trotz der Möglichkeit, vWF-Moleküle, denen die großen Multimeren fehlen, mit einem Kollagen-Bindungsassay sehr empfindlich zu erfassen, ist die Multimeren-Analyse für Patienten mit angeborenen, aber vor allem auch erworbenen Veränderungen des vWF ein wichtiger diagnostischer Test. Auch zur Bewertung von Faktor VIII-Gerinnungspräparaten ist die Ermittlung der Multimeren-Zusammensetzung von hoher Bedeutung.

Die vWF-Multimerenanalyse mittels SAGE-WISA ist Stand der Technik und von Metzner et al. (4) beschrieben worden. Das dort beschriebene Verfahren ist jedoch kompliziert und zeitaufwendig. Außerdem leidet es unter einer mangelnden Reproduzierbarkeit, was eine zuverlässige Quantifizierung der Multimeren-Banden erschwert. Hinzu kommen auch hier die Schwierigkeiten beim Gießen eines homogenen und von Verklumpungen freien Agarose-Gels sowie die Notwendigkeit, mit mehreren verschiedenen Agarose-Gelen zu arbeiten. Sowohl bei dem Verfahren von (4) als auch bei vergleichbaren Methoden (5, 6, 7) ist stets ein Trenngel und ein davon verschiedenes Sammelgel erforderlich. Zum Beispiel wird bei (4) ein 0,9%iges Agarose-Trenngel und ein 0,8%-iges Agarose-Sammelgel verwendet, während bei (5) ein 2%-iges Agarose-Trenngel und ein 1 %-iges Agarose-Sammelgel eingesetzt wird.

Hinzu kommt, dass für das Sammelgel und das Trenngel verschiedene Agarose-Typen vorgeschrieben werden. So wird in (4) eine HGT (= high gelling temperature)-Agarose für das Sammelgel und eine LGT (low gelling temperature)-Agarose für das Trenngel vorgeschrieben, während bei anderen bekannten Trennverfahren andere Agarose-Typen eingesetzt werden.

Eine zusätzliche Schwierigkeit taucht bei den herkömmlichen gelelektrophoretischen Trennungsverfahren häufig dadurch auf, dass das Agarose-Gel eine Tendenz zum Aufschwimmen zeigt. Dabei verliert das Agarose-Gel seine Bodenhaftung in der Elektrophoresekammer mit der Folge, dass sich schräge, verzerrte Spurmuster bilden oder es sogar zu einer elektrophoretischen Auswanderung von Proteinen aus dem Gel und somit zum Verlust von Proteinbanden kommt. Zwar kann das Aufschwimmen des Agarose-Gels durch eine katamaranartige Beschwerung des Gels verhindert werden, doch bewirkt das eine Kompression des Gels, was wiederum entweder die Bandentrennung beeinträchtigt oder die Zahl der verwendbaren Spuren pro Gel reduziert.

Die vorstehend genannten Schwierigkeiten können nun durch ein Verfahren zur qualitativen und quantitativen Bestimmung der Multimeren von Fibrinogen oder des von Willebrand-Faktors durch Gel-Elektrophorese gelöst werden, wenn man eine den von Willebrand-Faktor (vWF) oder Fibrinogen enthaltende Probe mittels eines kontinuierlichen, homogenen und von Verklumpungen freien Agarose-Gels submarin unter Kühlung auf 8°C bis 12°C elektrophoretisch auftrennt und die Multimeren-Banden nach einer Western Blot Analyse durch ein spezifisches Antikörper-Enzym-Konjugat auf der Blotting Membran immunchemisch oder durch einen geeigneten Farbstoff, vorzugsweise unter Blaufärbung, im Gel sichtbar macht.

Die erfindungsgemäß einzusetzenden homogenen und von Verklumpungen freien Agarose-Gele können zwar bei genügender Erfahrung auch selbst hergestellt werden, werden jedoch im allgemeinen käuflich erworben, zum Beispiel von den Firmen Elchrom oder Bio-Rad. Derartige Agarose-Gele, die bislang fast ausschließlich zur Auftrennung von DNA- oder RNA-Proben eingesetzt worden sind, weisen nur noch ein Trenngel mit einer einheitlichen Agarosekonzentra-tion auf und benötigen kein separates Sammelgel. Die Trennung wird dabei in folgender Weise durchgeführt:

### A. Auftrennung der vWF-Multimeren

Eingesetzt wurde für die Trennung der vWF-Multimeren ein Agarose-Gel mit einer Agarosekonzentration von 0,7 bis 1,8 Gewichtsprozent, vorzugsweise von 0,8 bis 1,2 Gewichtsprozent. Besonders vorteilhaft erwies sich für eine nachfolgende Western Blot Immunostain-Analyse (WISA) ein trägerfreies Agarose-Gel (z.B. von Bio-Rad). Für eine Coomassie Blue-Färbung erwies sich ein Agarose-Gel als besonders vorteilhaft, welches auf eine Trägerfolie aufgebracht war (z.B. von Elchrom Scientific AG), weil sich hierdurch die Agarose bei erhöhter Temperatur, bevorzugt zwischen 30°C und 50°C, besser trocknen und schneller mit Coomassie Blue, dem hierfür besonders bevorzugten Farbstoff, anfärben lässt.

Die Elektrophorese erfolgte submarin unter Kühlung auf 10°C in einer SEA 2.000 HIPER-Elektrophorese-Kammer (Elchrom Scientific, Cham, Schweiz) mit 0,8 - 1,5%-igen Agarose-Gelen und zum Beispiel einem Elektrophorese-Puffer aus Tris-Glycin-Puffer mit 0,1% SDS (Ansatz: 12,12 g Tris, 57,8 g Glycin und 2 g SDS wurden mit deionisiertem Wasser ad 2.000 ml aufgefüllt). Die Elektrophorese-Kammer wurde mit 2.000 ml Elektrophorese-Puffer beschickt. Dann wurde das Gel eingelegt und in einer Vorelektrophorese umequilibriert. Die Vorelektrophorese erfolgte bei 25 V / max. Watt / 0,5 h (mit laufender Umlaufpumpe, um ein schnelles Umäquilibrieren des Gels zu ermöglichen).

Zur Elektrophorese wurde das Gel so in die Elektrophorese-Kammer gelegt, dass die Auftragsstellen zur Kathode zeigten und das Gel während der gesamten Elektrophorese waagerecht und planparallel zum Spannungsfeld lag. Es wurde darauf geachtet, dass die Elektrophorese-Temperatur 8°C nicht unter- und 14°C nicht überschritt.

Der Probenauftrag erfolgte nach der Vorelektrophorese. Die Proben wurden in Probenpuffer ( bevorzugt z.B. SDS- und Bromphenolblau-haltiger Probenpuffer von Anamed oder Novex ) 3 Minuten im Wasserbad gekocht und jeweils 20 µl pro Spur in die Geltaschen aufgetragen.

Die Probenelektrophorese erfolgte bei 25 V / max. Watt / 0,5 h. Sie bewirkt das Eindringen der Proben in die Gel-Matrix.

Die Hauptelektrophorese erfolgte (unmittelbar im Anschluss an die Proben- elektrophorese) bei 50 V / max. Leistung, bis die Bromphenolblau-Front das Ende des Gels erreicht hatte.

Zur Coomassie Blue Färbung (SAGE-CB) wurden die Gele (bevorzugt Agarose-Gele auf Folie) zum Beispiel in einem Gemisch aus Ethanol, Essigsäure und Wasser (25/3/72; v/v) für 0,5 h fixiert. Dann wurden die Gele mit einer Lage von Filterpapieren unter Druck (zum Beispiel durch Auflegen einer gefüllten 10 I-Laborflasche) so lange ausgepresst, bis die Agarose matt erschien (ca. 2 - 3 h) und dann im Trockenschrank bei 40°C weiter getrocknet, bis die Agarose transparent erschien (ca. 1 - 2 h). Danach wurden die Gele unter leichtem Schaukeln bei 40°C über Nacht mit Coomassie Blue gefärbt (0,01% Serva Blue G, 6% Ammoniumnitrat, 0,2% Natriumacetat, 4 ml/l 65% HNO₃). Am nächsten Morgen wurden die Gele einmal kurz mit Wasser abgespült. Am Gelträger haftender Farbstoff wurde durch Abziehen der Trägerseite über ein mit Alkohol getränktes Filterpapier entfernt. Danach wurden die Gele erneut mit einer Lage von Filterpapieren unter Druck so lange ausgepresst, bis die Agarose matt erschien (ca. 1 h) und danach erneut im Trockenschrank bei 40°C weiter getrocknet, bis die Agarose transparent erschien (ca. 1 - 2 h).

Schließlich wurde das getrocknete Gel in eine Spezialfolie eingeschweißt bzw. laminiert, um es für die Auswertung und eine Langzeit-Dokumentation und Archivierung zu konservieren.

Zur Western Blot-Immunfärbung (SAGE-WISA), für die bevorzugt trägerfreie Agarose-Gele eingesetzt wurden, wurde der Blotpuffer am Analysentag morgens frisch angesetzt (35,6 g Na₂HPO₄ x 2 H₂O, 6,9 g NaH₂PO₄ x H₂O und 10 ml einer 20%igen wässrigen SDS Lösung wurden mit demineralisiertem Wasser ad 5.000 ml aufgefüllt), im Kühlschrank auf +12°C bis +14°C abgekühlt (Dauer ca. 3 h) und danach in die Blotkammer und die Reaktionsschalen gefüllt. Die Blotkammer-Kühlung wurde auf +8°C eingestellt. Die Membran (eine Nitrocellulose- oder - bevorzugt - PVDF-Membran, bevorzugt eine Immobilon-P 0,45 µm Transfer Membran von Millipore) und eine Pappe (zum Beispiel 165 x 65, Sarstedt AG) geschnitten auf 100 x 100 mm wurden vor dem Blot mindestens 0,5 h in Blotpuffer unter leichtem Schaukeln equilibriert.

Der gesamte Blot-Sandwich wurde in der mit Blotpuffer gefüllten Blotpufferschale wie folgt zusammengesetzt:
1. Blotrahmen (minus Rahmen)
2. dünne Schwamm-Folie
3. Pappe (100 x 100 mm)
4. Blot-Schutzmembran
5. Agarose-Gel (bevorzugter Weise wurden links und rechts des Gels jeweils zwei Filterstreifen (100 x 10 mm) angelegt), dann wurden die Luftblasen entfernt
6. Blotmembran (0,45 µm)
7. Pappe (100 x 100 mm)
8. Blotrahmen (plus Rahmen)

Luftbläschen unter dem Gel wurden mit den Fingern (Schutzhandschuhe) entfernt.

Der Blot erfolgte nach dem Fachmann an sich bekannten Verfahren unter Kühlung auf 8 ± 2°C und Rühren bei 20 V sowie max. Leistung über eine Dauer von 75 min.

Danach wurde der Blot Sandwich auseinander gebaut und die Blotmembran in eine Reaktionsschale mit Blocklösung gelegt (dabei musste die Seite, welche auf dem Gel lag, oben liegen). Die Blotmembran wurde 15 min. lang unter Schaukeln in RSA-Puffer blockiert (1 % Rinderserumalbumin (RSA) in Tris-gepufferter Kochsalzlösung, pH 8,0). Danach erfolgte die Immunfärbung.

### 1. Inkubation mit Erstantikörper (50 µl anti-vWF vom Kaninchen (zum Beispiel

Rabbit/Anti-Human/Humain, DAKO) in 0,5% RSA-Puffer) für 45 min.
2. Membran drei Mal mit demineralisiertem Wasser kurz abspülen.
3. 15 min. waschen mit 50 ml Tris-gepufferter Kochsalzlösung mit 0,5% RSA-Puffer
4. Inkubation mit Zweitantikörper (zum Beispiel 50 µl anti-Kaninchen IgG-AP von der Ziege (alkalische Phosphatase-markiert; Sigma) in 50 ml 0,5% RSA-Puffer) für 45 min.
5. Membran drei Mal mit demineralisiertem Wasser kurz abspülen.
6. 15 min. waschen mit 50 ml RSA-Puffer
7. Membran in Wanne mit 50 ml Substratlösung (2 Tabletten BCIP/NPT (Sigma) ad 50 ml 0,5% RSA-Puffer) 15 min. bei Raumtemperatur inkubieren
8. Abstoppen der Reaktion durch mehrmaliges Waschen mit demineralisiertem Wasser
9. Blotting-Membran auf die Heizplatte, die auf +40°C erwärmt wurde, legen, mit einer Pappe abdecken und die überschüssige Feuchtigkeit mit einem Rollenquetscher abrollen
10. Schließlich wurde die Blotmembran in eine Spezialfolie eingeschweißt bzw. laminiert, um sie für die Auswertung und eine Langzeit-Dokumentation und Archivierung zu konservieren.

Die Dokumentation des Blots oder des mit Coomassie Blue gefärbten Gels erfolgte als Original in laminierter Form oder durch Fotografieren oder Scannen.

Das gefärbte Gel (laminiert) oder die immungefärbte Blotmembran (laminert) wurde über einen Scanner eingelesen und mittels geeigneter Software densitometrisch ausgewertet (zum Beispiel mittels der "1-D-Elite", Version Image Master 4.10 Software von Pharmacia) und in üblicher Weise z.B. wie folgt bewertet:
- Die Anteile der Banden 1 - 5, 6 - 10 und 11 und höher wurden mittels der Auswertesoftware aufsummiert und als jeweiliger prozentualer Anteil auf die Gesamtspur bezogen, die als 100% zugrunde gelegt wurde.
- Der Anteil der Banden 11 und höher jeder Probe wurde als prozentualer Anteil auf die Banden 11 und höher des Standard-Humanplasmas (=100%) bezogen.

### B. Multimeren-Bestimmung von Fibrinogen

Eingesetzt wurde für die Trennung der Fibrinogen-Multimeren ein Agarose-Gel mit einer Agarosekonzentration von 1,6 bis 3,0 Gewichtsprozent, vorzugsweise von 1,8 bis 2,4 Gewichtsprozent. Besonders vorteilhaft erwies sich für eine nachfolgende Western Blot Immunostain-Analyse (WISA) ein trägerfreies Agarose-Gel (z.B. von Bio-Rad). Für eine Coomassie Blue-Färbung erwies sich ein Agarose-Gel als besonders vorteilhaft, welches auf eine Trägerfolie aufgebracht war (z.B. von Elchrom Scientific AG), weil sich hierdurch die Agarose bei erhöhter Temperatur, bevorzugt zwischen 30°C und 50°C, besser trocknen und schneller mit Coomassie Blue anfärben lässt.

Die Elektrophorese erfolgte submarin unter Kühlung auf 10°C in einer SEA 2.000 HIPER-Elektrophorese-Kammer (Elchrom Scientific, Cham, Schweiz) mit 1,6 - 3 Gew.%-igen, vorzugsweise 1,8 bis 2,4 Gew.-%-igen, Agarose-Gelen und z.B. einem Elektrophorese-Puffer aus Tris-Glycin mit 0,1% SDS (Ansatz: 12,12 g Tris, 57,8 g Glycin und 2 g SDS wurden mit deionisiertem Wasser ad 2.000 ml aufgefüllt). Die Elektrophorese-Zelle wurde mit 2.000 ml Elektrophorese-Puffer beschickt. Dann wurde das Gel eingelegt und in einer Vorelektrophorese umequilibriert. Die Vorelektrophorese erfolgte bei 25 V / max. Watt / 0,5 h (mit laufender Umlaufpumpe, um ein schnelles Umäquilibrieren des Gels zu ermöglichen).

Zur Elektrophorese wurde das Gel so in die Elektrophorese-Kammer gelegt, dass die Auftragsstellen zur Kathode zeigten und das Gel während der gesamten Elektrophorese waagerecht und planparallel zum Spannungsfeld lag. Es wurde darauf geachtet, dass die Elektrophorese-Temperatur 8°C nicht unter- und 14°C nicht überschritt.

Der Probenauftrag erfolgte nach der Vorelektrophorese. Die Proben wurden in Probenpuffer ( bevorzugt z.B. SDS- und Bromphenolblau-haltiger Probenpuffer von Anamed oder Novex ) 3 Minuten im Wasserbad gekocht und jeweils 20 µl pro Spur in die Geltaschen aufgetragen.

Die Probenelektrophorese erfolgte bei 25 V / max. Watt / 0,5 h. Sie bewirkt das Eindringen der Proben in die Gel-Matrix.

Die Hauptelektrophorese wurde unmittelbar im Anschluss an die Vorelektrophorese bei 50 V / max. Leistung / 0,5 h; danach 75 V / max. Watt, bis die Bromphenolblau-Front das Ende des Gels erreicht hatte, durchgeführt.

Zur Coomassie Blue Färbung wurden die Gele (vorzugsweise Agarose-Gele auf Folie z.B. von Elchrom Scientific) in einem Gemisch aus Ethanol, Essigsäure und Wasser (25/3/72; v/v) für 0,5 h fixiert. Dann wurden die Gele mit einer Lage von Filterpapieren unter Druck so lange ausgepresst, bis die Agarose matt erschien (ca. 2 - 3 h). Dann wurden die Gele im Trockenschrank bei 40°C weiter getrocknet, bis die Agarose transparent erschien (ca. 1 - 2 h). Danach wurden die Gele unter leichtem Schaukeln bei 40°C über Nacht mit Coomassie Blue gefärbt (0,01 % Serva Blue G, 6% Ammoniumnitrat, 0,2% Natriumacetat, 4 ml/l 65% HNO₃). Am nächsten Morgen wurden die Gele einmal kurz mit Wasser abgespült. Am Gelträger haftender Farbstoff wurde durch Abziehen der Trägerseite über ein mit Alkohol getränktes Filterpapier entfernt. Danach wurden die Gele erneut mit einer Lage von Filterpapieren unter Druck so lange ausgepresst, bis die Agarose matt erschien (ca. 1 h) und danach erneut im Trockenschrank bei 40°C weiter getrocknet, bis die Agarose transparent erschien (ca. 1 - 2 h).

Schließlich wurde das getrocknete Gel in eine Spezialfolie eingeschweißt bzw. laminiert, um es für die Auswertung und eine Langzeit-Dokumentation und Archivierung zu konservieren.

Zur Western Blot-Immunfärbung (SAGE-WISA), für die bevorzugt trägerfreie Agarose-Gele verwendet wurden, wurde der Blotpuffer am Analysentag morgens frisch angesetzt (35,6 g Na₂HPO₄ x 2 H₂O, 6,9 g NaH₂PO₄ x H₂O und 10 ml einer 20%igen wässrigen SDS Lösung wurden mit demineralisiertem Wasser ad 5.000 ml aufgefüllt), im Kühlschrank auf +12°C bis +14°C abgekühlt (Dauer ca. 3 h) und danach in die Blotkammer und die Reaktionsschalen gefüllt. Die Blotkammer-Kühlung wurde auf +8°C eingestellt. Die Membran (eine Nitrocellulose- oder - bevorzugt - PVDF-Membran, bevorzugt eine Immobilon-P 0,45 µm Transfer Membran von Millipore) und eine Pappe (z.B.165 x 65, Sarstedt AG), geschnitten auf 100 x 100 mm, wurden vor dem Blot mindestens 0,5 h in Blotpuffer unter leichtem Schaukeln equilibriert.

Der gesamte Blot-Sandwich wurde in der mit Blotpuffer gefüllten Blotpufferschale wie folgt zusammengesetzt:
1. Blotrahmen (minus Rahmen)
2. dünne Schwamm-Folie
3. Pappe (100 x 100 mm)
4. Blot-Schutzmembran
5. Agarose-Gel (bevorzugter Weise wurden links und rechts des Gels jeweils zwei Filterstreifen (100 x 10 mm) angelegt), dann wurden dieLuftblasen entfernt
6. Blotmembran (0,45 µm)
7. Pappe (100 x 100 mm)
8. Blotrahmen (plus Rahmen)

Luftbläschen unter dem Gel wurden mit den Fingern (Schutzhandschuhe) entfernt.

Der Blot erfolgte nach dem Fachmann an sich bekannten Verfahren unter Kühlung auf 8 ± 2 °C und unter Rühren bei 20 V bei max. Leistung über eine Dauer von 75 min.

Danach wurde der Blot Sandwich auseinander gebaut und die Blotmembran in eine Reaktionsschale mit Blocklösung gelegt (dabei musste die Seite, welche auf dem Gel lag, oben liegen). Die Membran wurde 15 min. lang unter Schaukeln in RSA-Puffer blockiert (1 % Rinderserumalbumin (RSA) in Tris-gepufferter Kochsalzlösung, pH 8,0). Danach erfolgte die Immunfärbung.
1. Inkubation mit Erstantikörper (50 µl anti-Fibrinogen vom Kaninchen (z.B. Rabbit/Anti-Human/Humain, DAKO) in 0,5% RSA-Puffer) für 45 min.
2. Membran drei Mal mit demineralisiertem Wasser kurz abspülen.
3. 15 min. waschen mit 50 ml Tris-gepufferter Kochsalzlösung mit 0,5% RSA-Puffer
4. Inkubation mit Zweitantikörper (zum Beispiel 50 µl anti-Kaninchen IgG-AP von der Ziege (alkalische Phosphatase-markiert; Sigma) in 50 ml 0,5% RSA-Puffer) für 45 min.
5. Membran drei Mal mit demineralisiertem Wasser kurz abspülen.
6. 15 min. waschen mit 50 ml RSA-Puffer
7. Membran in Wanne mit 50 ml Substratlösung (2 Tabletten BCIP/NPT (Sigma) ad 50 ml 0,5% RSA-Puffer) 15 min. bei Raumtemperatur inkubieren
8. Abstoppen der Reaktion durch mehrmaliges Waschen mit demineralisiertem-Wasser
9. Blotting-Membran auf die Heizplatte, die auf +40°C erwärmt wurde, legen, mit einer Pappe abdecken und die überschüssige Feuchtigkeit mit einem Rollenquetscher abrollen
10. Schließlich wurde die Blotmembran in eine Spezialfolie eingeschweißt bzw. laminiert, um sie für die Auswertung und eine Langzeit-Dokumenta- tion und Archivierung zu konservieren.

Die Dokumentation des Blots oder des mit Coomassie Blue gefärbten Gels erfolgte als Original in laminierter Form oder durch Fotografieren oder Scannen.

Das gefärbte Gel (laminiert) oder die immungefärbte Blotmembran (laminert) wurde über einen Scanner eingelesen und mittels geeigneter Software densitometrisch ausgewertet (zum Beispiel mittels der "1-D-Elite", Version Image Master 4.10 Software von Pharmacia):

### C. Auswertung der Auftrennung der vWF-Multimeren

### Beispiel 1

### SAGE-WISA einer Verdünnungsreihe von Standard Humanplasma in einem 1% Agarose-Fertiggel von Bio-Rad.

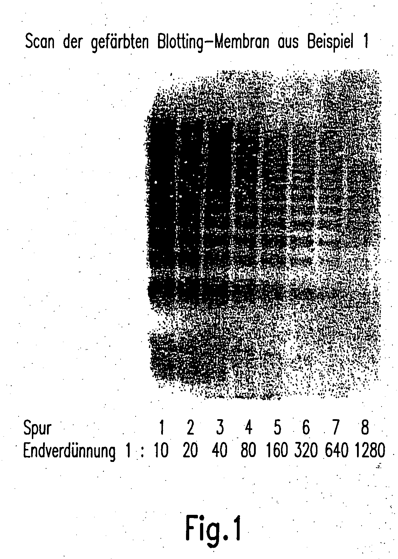

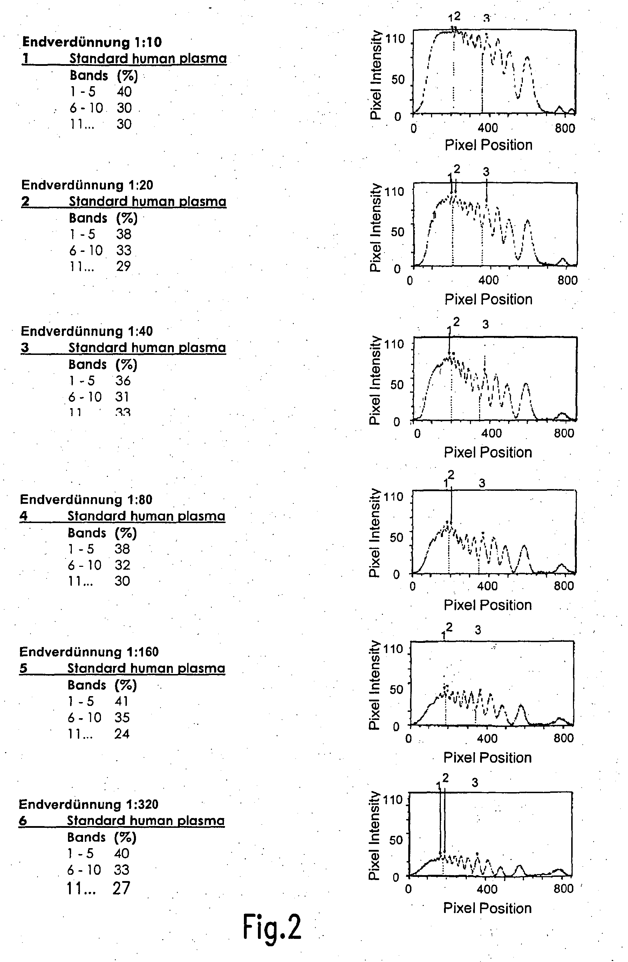

Standard-Humanplasma (Dade Behring) wurde mit demineralisiertem Wasser in einer geometrischen Verdünnungsreihe verdünnt. Die Verdünnungen wurden 1 : 1 (v/v) mit Probenpuffer versetzt, so dass sich die in Abb. 1 ausgewiesenen Endkonzentrationen ergaben. Die Proben wurden in Probenpuffer ( bevorzugt z.B. SDSund Bromphenolblau-haltiger Probenpuffer von Anamed oder Novex ) 3 Minuten im Wasserbad gekocht und jeweils 20 µl pro Spur in die Geltaschen aufgetragen. Der Scan der gefärbten Blotting-Membran ist in Abb. 1 gezeigt, seine densitometrische Auswertung in Abb. 2. Die Quantifizierung der Peakgruppen erfolgte analog (4).

### Beispiel 2

### SAGE-WISA einer Verdünnungsreihe von Haemate-HS® in einem 1% Agarose Fertiggel von Bio-Rad.

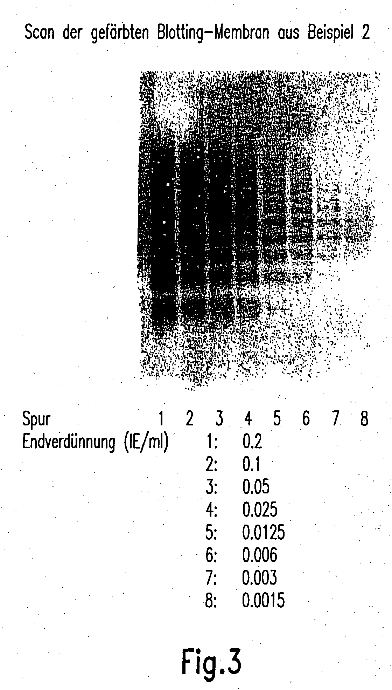

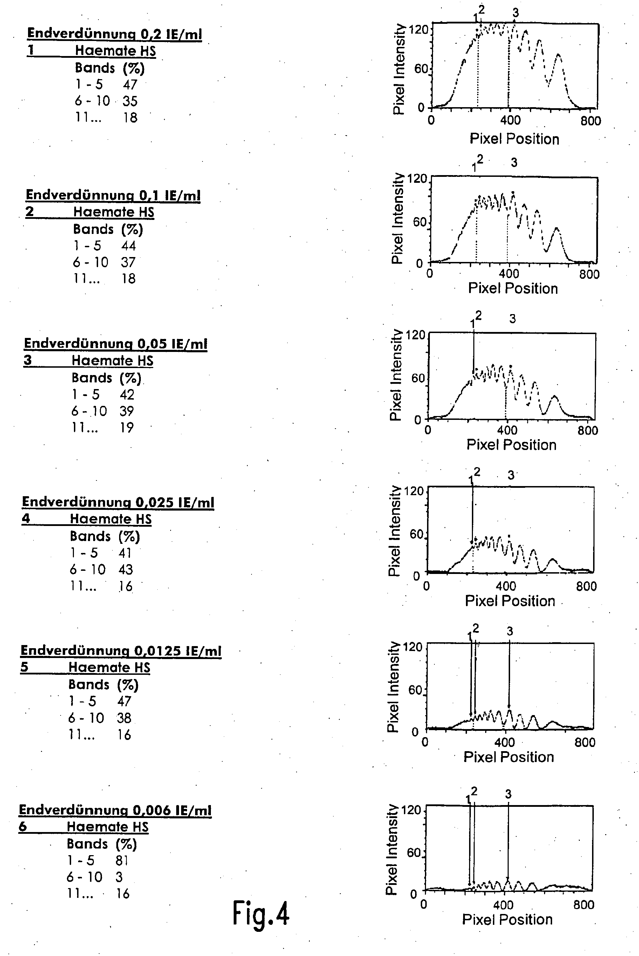

Haemate-HS® (Aventis Behring) wurde mit demineralisiertem Wasser in einer geometrischen Verdünnungsreihe verdünnt. Die Verdünnungen wurden 1 : 1 (v/v) mit Probenpuffer versetzt, so dass sich die in Abb. 3 ausgewiesenen Endkonzentrationen ergaben. Die Proben wurden 3 min. im Wasserbad gekocht und in jeweils 20 µl pro Spur in die Geltaschen aufgetragen. Der Scan der gefärbten Blotting-Membran ist in Abb. 3, seine densitometrische Auswertung in Abb. 4 gezeigt. Die Quantifizierung der Peakgruppen erfolgte analog (4).

### Beispiel 3

### SAGE-CB von Humate-P® und Haemate-HS® in einem 1 % Agarose-Fertiggel der Elchrom Scientific AG

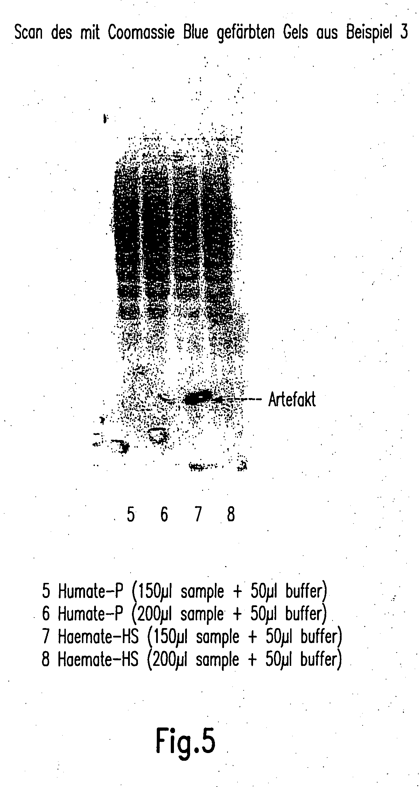

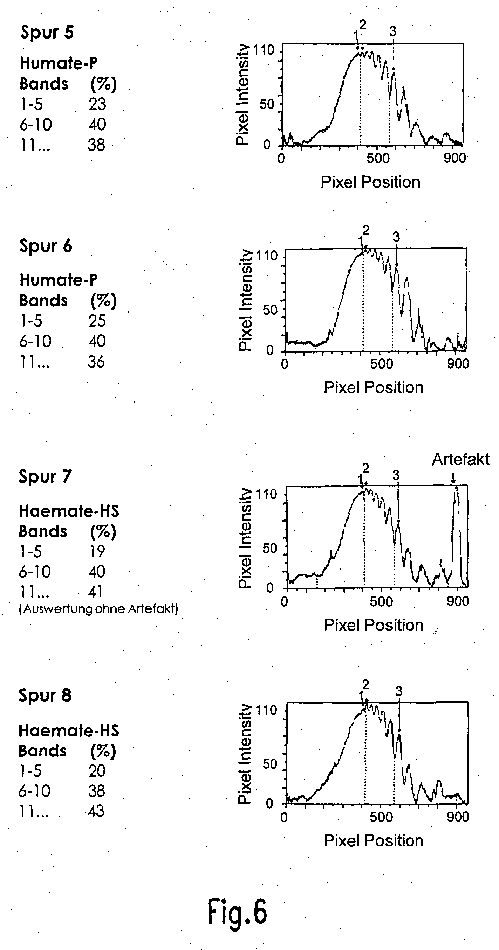

Humate-P® und Haemate-HS@ wurden mit Probenpuffer auf die in Abb. 5 gezeigten Konzentrationen verdünnt, 3 min im Wasserbad gekocht und jeweils 20 µl pro Spur in die Geltaschen aufgetragen. Der Scan des gefärbten Gels ist in Abb.5, seine densitometrische Auswertung in Abb.6 gezeigt. Die Quantifizierung der Peakgruppen erfolgte analog (4).

Ein Vergleich der densitometrischen Auswertungen der vWF-Proben aus Abb.4 (Blot-Membran) und Abb.6 (Blaufärbung im Gel) zeigt, dass der prozentuale Anteil der Banden 11 und höher im Western Blot ca. 18% beträgt, in der Blaufärbung jedoch mit ca. 40% praktisch doppelt so hoch ist. Dieser Befund unterstreicht die aus (8) bekannte Beobachtung, dass die hochmolekularen Banden schlechter geblottet werden. Perutelli hat diese Schwachstelle des Western Blots durch eine Direktfärbung der Multimerenbanden im Gel mit einem radioaktiven Antikörperkonjugat (Autoradiographie oder Luminographie) überwunden. Demgegenüber hat die erfindungsgemäß verwendete Blaufärbung den Vorteil, dass sie völlig gefahrlos, wenn auch weniger sensitiv ist.

### D. Auswertung der Auftrennung der Fibrinogen-Multimeren

### Beispiel 4

### SAGE-CB von Fibrinogen in einem 2% Agarose-Fertiggel der Elchrom Scientific AG.

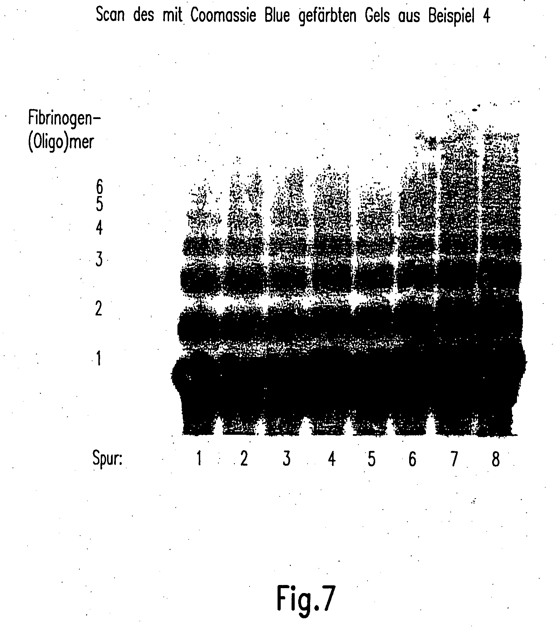

Die Proben wurden 1 : 1 (v/v) mit Probenpuffer versetzt, 3 min. im Wasserbad gekocht und jeweils 20 µl (≈ 360 µg Fibrinogen; 18 mg/ml) pro Spur in die Gel taschen aufgetragen. Der Scan des mit Coomassie-Blue gefärbten Gels ist in Abb. 7 gezeigt.

### Beispiel 5

### SAGE-WISA von Fibrinogen in einem 2% Agarose-Fertiggel von Elchrom Scientific.

Die Fibrinogen-Proben (90g/l) wurden 1:100 (v/v) mit demineralisiertem Wasser und danach 1:12 (v/v) mit Probenpuffer verdünnt, 3 min im Wasserbad gekocht und jeweils 20 µl pro Spur in die Geltaschen aufgetragen.

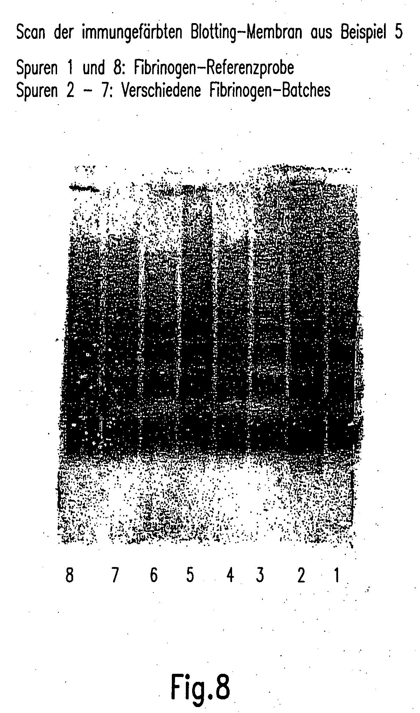

Der Scan der immungefärbten Blotting-Membran ist in Abb.8 gezeigt. Dabei wurde zum Blot das Agarose-Gel von der Trägerfolie abgelöst.

### Beispiel 6

### SAGE-WISA von Humate-P® in einem 2% Agarose-Fertiggel von Elchrom Scientific.

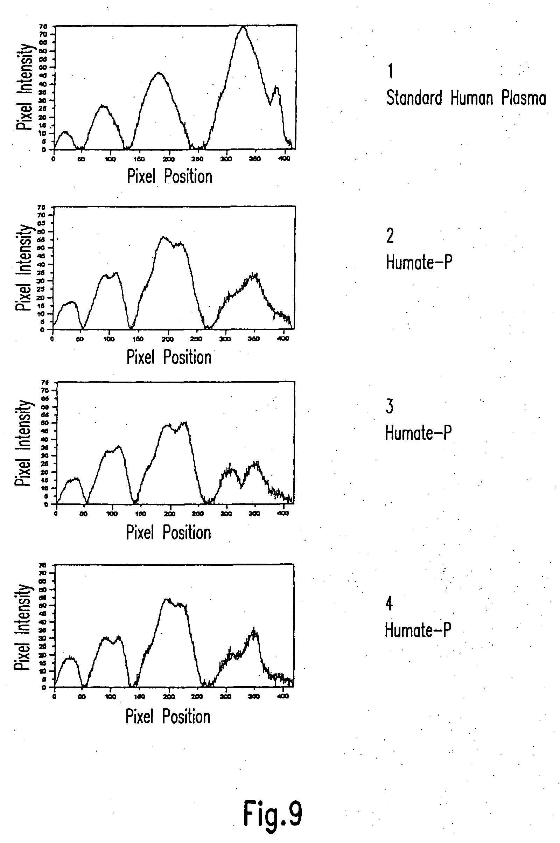

Die Humate-Probe wurde analog Beispiel 2 mit demineralisiertem Wasser vorverdünnt und mit Probenpuffer 1 : 1 versetzt, so dass sich die in Abb. 3, Spur 2 ausgewiesene Endkonzentration ergab (0.1 IE/mL). Die Humate-Probe wurde 3 min im Wasserbad gekocht und jeweils 20 µl pro Spur in die Geltaschen 2 bis 7 gegen Standard Human Plasma (Spur 1 und Spur 8) in einem 2%igen Agarose-Gel von Elchrom Scientific vermessen, wobei die Hauptelektrophorese gegenüber der Vermessung im 1%igen Gel von 3 auf 4,5 Stunden erhöht wurde. Blot und Gelfärbung erfolgten analog Beispiel 2. Der Scan der gefärbten Blotting-Membran und die densitometrische Auswertung des niedermolekularen Bereichs der Spuren 1 bis 4 ist in Abb. 9 gezeigt. Dabei wurde zum Blot das Agarose-Gel von der Trägerfolie abgelöst. Gegenüber Standard Human Plasma zeigten die niedermolekularen Multimerenbanden von Humate-P eine Feinstruktur (Aufspaltung in Doppelbanden).

### Literaturverzeichnis:

1. Connaghan, D.G., Frahcis, C.W., Lane, D.A.; Marder, V.J. (1985) Specific identification of fibrin polymers, fibrinogen degradation products and crosslinked fibrin degradation products in plasma and serum with a new sensitive technique. Blood 65 (3) 589 - 97.
2. Proietti, A.B.; McGuire, M.; Bell, W. (1990) Specific identification of fibrin(ogen) degradation products in plasma and serum usinig blotting and peroxidase labeled antiserum. American Journal of Hematology 34 (4) 270 - 4.
3. Raines, G.; Aumann. H,; Sykes, S.; Street A (1990) Multimeric analysis of von Willebrand factor by molecular sieving electrophoresis in sodium dodecyl sulphate agarose gel. Thrombosis Research 60 (3) 201 - 12.
4. Metzner, H.J.; Hermentin, P.; Cuesta-Linker, T.; Langner, S.; Müller, H.-G. und Friedebold, J. (1998) "Characterization of factor VIII/von Willebrand factor concentrates using a modified method of von Willebrand factor multimer analysis" Haemophilia (1998), 4 (Suppl. 3), 25 - 32.
5. Coon und Bayer (1995) Clin. Appl. Thrombosis/Hemostasis 1, 31 - 3
6. Tatewaki et al., (1989) Thromb. Res. 56, 191 - 9
7. Ruggeri und Zimmermann (1981) Blood 57: 1140 - 3
8. Perutelli, P., Haematologica 2002; 87:223-224

## Patentansprüche

1. Verfahren zur qualitativen und quantitativen Bestimmung der Multimeren von multimeren bildenden therapeutischen Proteinen durch Gelelektrophorese, **dadurch gekennzeichnet, dass** man eine den von Willebrand-Faktor (vWF) oder Fibrinogen enthaltende Probe mittels eines kontinuierlichen, homogenen und von Verklumpungen freien Agarose-Gels submarin elektrophoretisch auftrennt und die Multimerenbanden nach einer Western Blot-Analyse durch ein spezifisches Antikörper-Enzym-Konjugat auf der Blotting-Membran immunchemisch oder durch einen geeigneten Farbstoff, vorzugsweise unter Blaufärbung, im Gel sichtbar macht.

2. Verfahren nach Anspruch 1, **dadurch gekennzeichnet, dass** das multimeren bildende therapeutische Protein Fibrinogen ist.

3. Verfahren nach Anspruch 1, **dadurch gekennzeichnet, dass** das multimeren bildende therapeutische Protein von Willebrand Faktor (vWF) ist.

4. Verfahren nach Anspruch 2, **dadurch gekennzeichnet, dass** für die Trennung der Fibrinogen-Multimeren ein Agarose-Gel mit einer Agarose-Konzentration von 1,6 - 3 Gew.%, vorzugsweise von 1,8 - 2,4 Gew.-% eingesetzt wird.

5. Verfahren nach Anspruch 3, **dadurch gekennzeichnet, dass** für die Trennung der vWF-Multimeren ein Agarose-Gel mit einer Agarose-Konzentration von 0,7 - 1,8 Gew.-%, vorzugsweise von 0,8 - 1,2 Gew.-% eingesetzt wird.

6. Verfahren nach den Ansprüchen 1 bis 5, **dadurch gekennzeichnet, dass** die Gelelektrophorese bei Temperaturen zwischen 6°C und 14°C, vorzugsweise zwischen 8°C und 12°C, durchgeführt wird.

7. Verfahren nach den Ansprüchen 1 bis 6, **dadurch gekennzeichnet, dass** zum Anfärben der Multimerenbanden auf der Blotting-Membran eine Immunfärbung eingesetzt wird.

8. Verfahren nach den Ansprüchen 1 bis 6, **dadurch gekennzeichnet, dass** zur Blaufärbung der Multimerenbanden im Gel der Farbstoff Coomassie Blue eingesetzt wird.

9. Verfahren nach den Ansprüchen 1 bis 8, **dadurch gekennzeichnet, dass** für die Blaufärbung im Gel ein auf eine Trägerfolie aufgetragenes Agarose-Gel eingesetzt wird.

10. Verfahren nach den Ansprüchen 1 bis 8, **dadurch gekennzeichnet, dass** für die Immunfärbung auf der Blotting-Membran ein Trägerfolien-freies Agarose-Gel eingesetzt oder die Trägerfolie vor dem Blot-Vorgang vom Gel entfernt wird.

11. Verfahren nach den Ansprüchen 1 bis 10, **dadurch gekennzeichnet, dass** die Banden densitometrisch quantifiziert werden.

12. Verfahren nach Anspruch 11, **dadurch gekennzeichnet, dass** die Banden nach Blaufärbung des Gels quantifiziert werden.

13. Verfahren nach Anspruch 11, **dadurch gekennzeichnet, dass** die Banden nach Immunfärbung der Blotting-Membran quantifiziert werden.

14. Verfahren nach Anspruch 9, **dadurch gekennzeichnet, dass** das Gel nach der Färbung durch Laminierung konserviert wird.

15. Verfahren nach Anspruch 10, **dadurch gekennzeichnet, dass** die Blotting-Membran nach der Immunfärbung durch Laminierung konserviert wird.
